# EUROPEAN PATENT SPECIFICATION

(11) **EP 4 649 423 B1**
(45) Date of publication and mention of the grant of the patent: **25.11.2026**
(21) Application number: 23916545.9
(22) Date of filing: 25.08.2023
(51) Int. Cl.: G06N 3/04, G16H 40/60, G06N 3/045, G06N 3/08, G16H 10/40, G16H 30/20, G16H 40/40, G16H 40/63, G16H 40/67

(54) **COMPREHENSIVE ACTION PLAN RECOMMENDATIONS FOR MEDICAL MACHINES USING TRANSFORMER NETWORKS**
UMFASSENDE AKTIONSPLANEMPFEHLUNGEN FÜR MEDIZINISCHE MASCHINEN MIT TRANSFORMATORNETZWERKEN
RECOMMANDATIONS DE PLAN D'ACTION COMPLÈTES ET DÉTAILLÉES POUR MACHINES MÉDICALES UTILISANT DES RÉSEAUX TRANSFORMEURS

(30) Priority: 13.01.2023 US 202363479775 P
(43) Date of publication of application: 19.11.2025
(73) Proprietor: Siemens Healthcare Diagnostics, Inc., Tarrytown, NY 10591 (US)
(72) Inventor: FARRI, Oladimeji, Upper Saddle River, New Jersey 07458 (US); KUMAR KARN, Sanjeev, Plainsboro, New Jersey 08536 (US)
(74) Representative: Dr. Müller Patentanwälte
(86) International application number: PCT/US2023/072885
(87) International publication number: WO 2024/151323

(56) References cited:
- US-A1- 2019 034 591
- US-A1- 2020 337 648
- US-A1- 2020 337 648
- US-A1- 2022 059 200
- US-A1- 2022 245 799
- LING CHEN ET AL: "Fault Text Classification of Rotating Machine Based BERT", 2021 33RD CHINESE CONTROL AND DECISION CONFERENCE (CCDC), IEEE, 22 May 2021 (2021-05-22), pages 6744 - 6750, XP034031327, DOI: 10.1109/CCDC52312.2021.9602286
- AHMED SABEEN; NIELSEN IAN E.; TRIPATHI AAKASH; SIDDIQUI SHAMOON; RAMACHANDRAN RAVI P.; RASOOL GHULAM: "Transformers in Time-Series Analysis: A Tutorial", CIRCUITS, SYSTEMS AND SIGNAL PROCESSING., CAMBRIDGE, MS., US, vol. 42, no. 12, 25 July 2023 (2023-07-25), US , pages 7433 - 7466, XP037947768, ISSN: 0278-081X, DOI: 10.1007/s00034-023-02454-8

## Description

### TECHNICAL FIELD

The present invention relates generally to comprehensive action plan recommendations for medical machines, and in particular to comprehensive action plan recommendations for medical machines using transformer networks.

### BACKGROUND

Medical machines are often used for the diagnosis, monitoring, or treatment of medical conditions. Examples of such medical machines include diagnostic imaging machines and laboratory diagnostic machines. To maintain such medical machines, a set of automatic tests or checks are frequency performed. Each automatic test results in a time series representing results of the test.

Failed automatic tests are typically accompanied by an event log and/or alphanumeric codes that describe the error associated with the failure, as well as potential troubleshooting steps. Such troubleshooting steps are generated by leveraging years of human expertise and experience, often with the underlying assumption that events/issues are independent or occur serially. However, this is rarely the case when operational issues occur with complex medical machines. Therefore, such troubleshooting steps are often inadequate or inappropriate to completely resolve complex issues and prevent unnecessary downtime. US 2022/245799 A1 relates with troubleshooting of medical devices and corresponding resolution.

### BRIEF SUMMARY OF THE INVENTION

In accordance with one or more embodiments, systems and methods for generating a predicted event log are provided. A time series resulting from a test of a medical machine is received. The time series is encoded using an encoder of a transformer network. A predicted event log for the medical machine is generated based on the encoded time series using a decoder of the transformer network. The predicted event log comprises predicted events and associated action plan recommendations. The predicted event log is output.

In one embodiment, the encoder comprises one or more encoder blocks each comprising a multi-head self-attention sublayer. The time series is encoded by generating query, key, and value matrices based on a vector representation of the time series. An attention matrix is computed for each attention head of the multi-head self-attention sublayers of the one or more encoder blocks based on the query, key, and value matrices. The attention matrices for all attention heads are combined to generate the encoded time series.

In one embodiment, the transformer network is trained using training event logs. The decoder comprises one or more decoder blocks each comprising a multi-head encoder-decoder attention sublayer each comprising a multi-head encoder-decoder attention sublayer parameterized by a key matrix and a value matrix computed based on the encoded time series and query matrix computed based on an event log embedding matrix learned during training. The one or more decoder blocks each further comprise a masked multi-head self-attention sublayer for learning an event log embedding matrix based on the training event logs. The masked multi-head self-attention sublayers mask tokens in a vector representation of the training event logs.

In one embodiment, the time series is preprocessed to link sensors of the medical machine.

In one embodiment, the medical machine comprises at least one of a medical imaging machine or a medical laboratory machine.

These and other advantages of the invention will be apparent to those of ordinary skill in the art by reference to the following detailed description and the accompanying drawings.

### BRIEF DESCRIPTION OF THE DRAWINGS

Figure 1 shows a method for generating action plan recommendations for medical machines, in accordance with one or more embodiments;
Figure 2 shows a workflow for generating action plan recommendations for medical machines, in accordance with one or more embodiments;
Figure 3 shows an exemplary artificial neural network that may be used to implement one or more embodiments;
Figure 4 shows a convolutional neural network that may be used to implement one or more embodiments; and
Figure 5 shows a high-level block diagram of a computer that may be used to implement one or more embodiments.

### DETAILED DESCRIPTION

The present invention generally relates to methods and systems for comprehensive action plan recommendations for medical machines using transformer networks. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system.

Embodiments described herein provide for a transformer neural network for automatically generating comprehensive action plan recommendations for medical machines, such as, e.g., medical imaging machines and laboratory diagnostic machines. The transformer network receives as input a time series and generates as output a predicted event log comprising predicted events and associated action plan recommendations. Advantageously, embodiments described herein provide for an increased likelihood of appropriately solving complex operational issues for the medical machines.

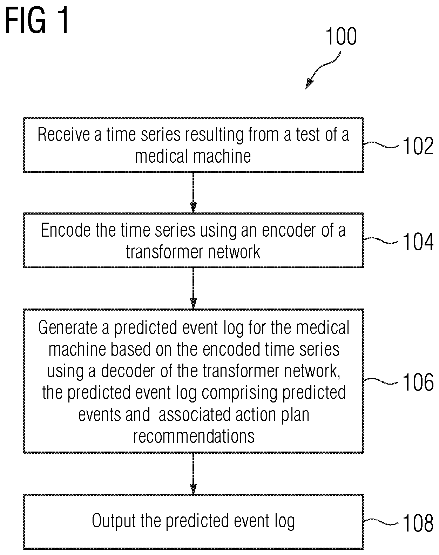

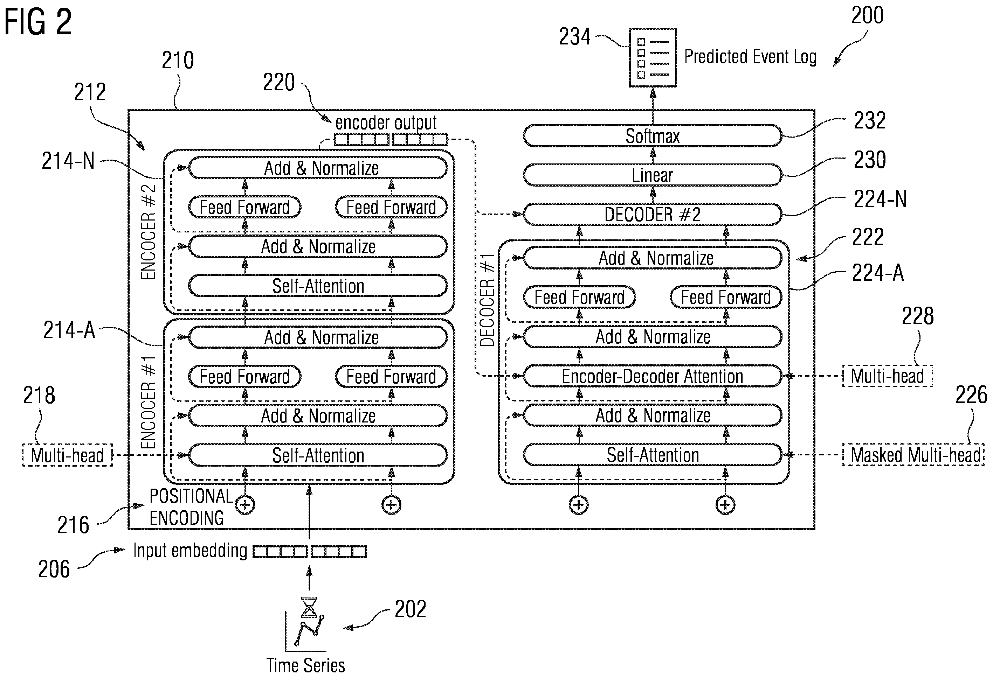

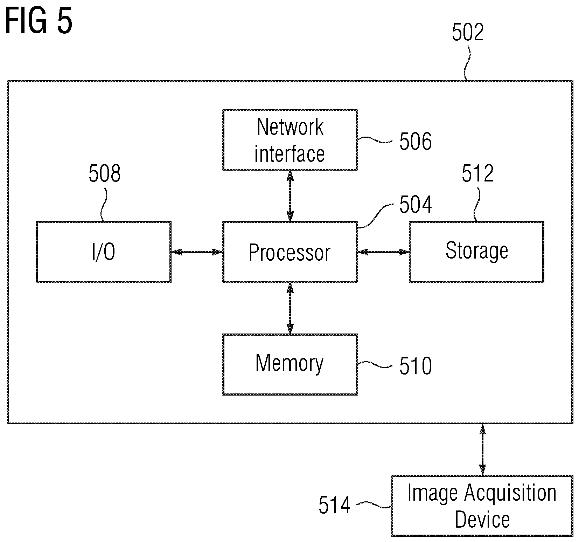

Figure 1 shows a method 100 for generating action plan recommendations for medical machines, in accordance with one or more embodiments. The steps of method 100 of Figure 1 may be performed by one or more suitable computing devices, such as, e.g., computer 502 of Figure 5. Figure 2 shows a workflow 200 for generating action plan recommendations for medical machines, in accordance with one or more embodiments. Figure 1 and Figure 2 will be described together.

At step 102 of Figure 1, a time series resulting from a test of a medical machine is received. In one example, as shown in workflow 200 of Figure 2, the time series is time series 202.

The test may be, for example, an automatic test (or automatic check) automatically performed by the medical machine. The automatic test may be periodically performed by the medical machine, e.g., at predetermined time intervals or at a predetermined schedule as part of a prescriptive maintenance plan. However, the test may be a manual test or any other suitable test of the medical machine. As a result of the test, the medical machine generates the time series. The time series represents results of the test, for example, as a series of numbers.

The medical machine may be any suitable medical machine. In one example, the medical machine is a medical imaging machine, such as, e.g., an MRI (magnetic resonance imaging) scanner, a CT (computed tomography) scanner, an ultrasound scanner, an x-ray scanner, or any other suitable medical imaging machine. In another example, the medical machine is a medical laboratory machine, such as, e.g., a hematology analyzer, a centrifuge, an incubator, a cell counter, a microplate reader, a PCR (polymerase chain reaction) machine, or any other suitable laboratory machine. Other examples of the medical machine include handheld (point of care) analyzers and radiotherapy machines.

The time series may be, for example, received directly from the medical machine as the test is run and completed, received by loading a stored time series (e.g., generated from a previously completed test) from a storage or memory of a computer system, or received from a remote computer system.

At step 104 of Figure 1, the time series is encoded using an encoder of a transformer network. In one example, as shown in workflow 200, the encoder is encoder 212 of transformer network 210 of Figure 2. However, the encoder may be of any suitable architecture.

Encoder 212 comprises one or more encoder blocks 214-A, ..., 214-N (collectively referred to as encoder blocks 214). In one embodiment, encoder 212 comprises 12 to 24 encoder blocks 214. However, encoder 212 may comprise any suitable number of encoder blocks 214. As shown in Figure 2, each encoder block 214 comprises a self-attention sublayer, a first add and normalize sublayer, feed forward sublayers, and a second add and normalize sublayer.

First encoder block 214-A of encoder 212 receives input embedding 206 of time series 202 as input. Input embedding 206 is a vector representation of time series 202 comprising vectors *h*₁ through *hₙ*. In one embodiment, the vector representation of time series 202 may be concatenated with positional encoding 216 to generate input embedding 206 to facilitate the encoding of time series 202 temporally. The encoded time series output from each encoder block 214 is passed as input to the next encoder block 214 to generate encoder output 220. For example, as shown in Figure 2, an encoded representation of input embedding 206 is output from first encoder block 214-A and passed as input to second encoder block 214-N, which outputs the encoded representation of input embedding 206 as encoder output 220.

In one embodiment, the self-attention sublayer of each encoder block 214 comprises a multi-head self-attention layer with multiple attention heads parameterized by query (Q), key (K), and value (V) matrices. Each vector h₁ through hₙ of input embedding 206 is linearly transformed in query vector qᵢ, key vector kᵢ, and value vector *vᵢ.* All vectors *qᵢ, kᵢ,* and *vᵢ* are packed into the Q, K, and V matrices respectively. Q and K matrices are respectively initialized with random weights *W^{Q}* and *W^{K}* and a SoftMax function is applied to the product of Q and K (scaled using the square root of the dimension of key vector k) to calculate the weight *W^{V}* for the V matrix. The attention matrix *Attention(Q, K, V)* for the self-attention sublayer is the summation of *VW^{V}* from all possible combination pairs of vectors from Q and K matrices, as shown in Equation (1): $Attention \left(Q, K, V\right) = softmax \left(\frac{Q ⋅ {K}^{T}}{\sqrt{{d}_{k}}}\right) V$ (1) where dₖ is the dimension of the key matrix. The attention matrices from all heads of all encoder blocks 214 are then combined (e.g., concatenated) to produce encoder output 220 representing an encoding of time series 202.

In the 'add & normalize' sublayers, the 'add' aspect represents a residual connection between the self-attention sublayers across different blocks (in the first add & normalize sublayer, after the feed forward network), and between the self-attention sublayer and the simple position-wise fully connected feed forward network within a block (in the second add & normalize sublayer, between the self-attention sublayer and the feed forward network). These connections force the input of the subsequent sublayer to include the original vectors (h₁ through hₙ) alongside the output of the previous sublayer. The 'normalize' aspect represents a function to transform all outputs from the self-attention layers into a specific dimension (e.g., d_{model} = 512).

In one embodiment, time series 202 may be preprocessed to link sensors of the medical machine with corresponding values. Each sensor-specific time series is tokenized and transformed to the source sentence. In particular, given that time series 202 is represented as sequence of tokens, an additional token specific to each sensor is included before generating the vectors h₁ through hₙ as inputs into the self-attention sublayer of first encoder block 214-A. Therefore, the resulting vectorized sequence (hᵢⱼ) is implicitly linked to a specific machine-related sensor due to the sensor-specific token embedded in the vector.

At step 106 of Figure 1, a predicted event log for the medical machine is generated based on the encoded time series using a decoder of the transformer network. The predicted event log comprises predicted events and associated action plan recommendations. In one example, as shown in workflow 200 of Figure 2, decoder 222 of transformer network 210 generates predicted event log 234 based on encoder output 220.

Decoder 222 comprises one or more decoder blocks 224-A, ..., 224-N (collectively referred to as decoder blocks 224). In one embodiment, decoder 222 comprises the same number of decoder blocks 224 as encoder 212 has encoder blocks 214. As shown in Figure 2, each decoder block 224 comprises a self-attention sublayer, a first add and normalize sublayer, an encoder-decoder attention sublayer, a second add and normalize sublayer, feed forward sublayers, and a third add and normalize sublayer. The self-attention sublayer and the first and the second add & normalize sublayers are similar in operation as in encoder blocks 214. The third add & normalize sublayer (between the self-attention and the encoder-decoder attention sublayers) is similar in operation as in encoder blocks 214, except it represents a residual connection between the self-attention and the encoder-decoder attention sublayers.

First decoder block 224-A of decoder 222 receives encoder output 220 as input. The decoded representation of encoder output 220 output from each decoder block 224 is passed as input to the next decoder block 224 to generate predicted event log 234. For example, as shown in Figure 2, the decoded representation of encoder output 220 is output from first decoder block 224-A and passed as input to second decoder block 224-N, which outputs the predicted event log 234.

In one embodiment, the encoder-decoder attention sublayer of each decoder block 224 comprises a multi-head encoder-decoder attention sublayer 228. Similar to multi-head attention sublayer 218 in encoder 212, the multi-head encoder-decoder attention sublayer 228 is parameterized by Q, K, and V matrices to compute the attention matrix for each attention head in the multi-head encoder-decoder attention sublayer 228. As shown in Figure 2, multi-head encoder-decoder attention sublayer 228 receives encoder output 220 from encoder 212 as input and the K and V matrices are computed based on encoder output 220. However, unlike multi-head attention sublayer 218, the Q matrix is computed from an event log embedding matrix (learned during the training stage by masked multi-head self-attention sublayer 226) rather than from the input embedding 206 of time series 202. In other words, the multi-head encoder-decoder attention sublayer 228 allows the sequences learned from training event logs to 'attend' to the sequences from time series 202, unlike the self-attention sublayer in which time series 202 (in an encoder block 214) or training event logs (in a decoder block 224 during the training stage) 'attends' only to itself. In this manner, decoder 222 learns to attend to related time series and event log tokens over several training epochs.

In one embodiment, the self-attention sublayer of each decoder block 224 comprises a masked multi-head self-attention sublayer 226. Aside from first decoder block 224-A, each masked multi-head self-attention sublayer 226 receives the decoded representation of a predicted event log as input. The attention matrices from all the heads of all decoder blocks 224 are concatenated to fully represent the decoded representation of the predicted event log 234.

The decoded representation of the predicted event log 234 output from multi-head encoder-decoder attention sublayer 228 is transferred to feed forward neural network sublayers and an add and normalize sublayer to further learn the optimal weights via backpropagation and to ensure residual connections and layer normalization for computational efficiency. In this embodiment, the feed forward and the add and normalize sublayers are present in both the encoder and decoder blocks.

The final decoded representation of the predicted event log 234 is a representation of the predicted event log 234 attended to the input time series 202 and is passed from decoder block 224-N to a linear layer 230 and a softmax layer 232. Softmax layer 232 generates the final predicted event log 234 based on the decoded representation output from decoder block 224-N. At softmax layer 232, the next token is predicted based on the previous token to generate the predicted event log 234. In this manner, softmax layer 232 computes the probability over the vocabulary of event log tokens in the decoded representation output from decoder block 224-N such that the model can progressively predict the next token based on those most relevant to time series 202. Ultimately, as time series 202 is continuously fed into the model, the combination of predicted event log tokens would be presented in predicted event log 234 as predicted events and associated action plans recommendations.

In one example, the predicted event log comprises the following predicted events and associated action plan recommendations: "In 48hrs, machine part may have lamp intensity value on one or more of the wavelengths as low and below X value, the reference channel as below Y or a wavelength as above saturation of Z. The first recommendation is to replace lamp."

At step 108 of Figure 1, the predicted event log is output. For example, the predicted event log can be output by displaying the predicted event log on a display device of a computer system, storing the predicted event log on a memory or storage of a computer system, or by transmitting the predicted event log to a remote computer system. Advantageously, the predicted event log enables users to perform prescriptive maintenance according to the action plans recommendations to proactively address the predicted events and avoid operational issues.

Transformer network 210 is trained during a prior offline training stage. In one embodiment, the transformer network 210 is pretrained using a sequence-to-sequence approach and fine-tuned based on a prescriptive maintenance task. A collection of machine-derived training time series and corresponding training event logs are utilized as the training data. The transformer network 210 may be trained using, for example, an MLM (masked language modeling) loss. Once trained, the trained transformer network 210 is applied during an inference or testing stage, for example, to perform steps 104 and 106 of Figure 1. At test time, the fine-tuned transformer network 210 would receive time series 202 from a medical machine as input, with the object being to output a set of predicted event logs 234 that identify potential issues/events and associated action plan recommendations for the same medical machine at future points in time.

Similar to time series 202, the training time series represent results of a test of a medical machine. The training event logs comprise a description and/or alphanumeric codes of errors or events experienced during the test and may comprise troubleshooting steps for resolving the errors. During the training stage, multi-head self-attention sublayer 218 of first encoder block 214-A receives an input embedding of training time series as input and masked multi-head self-attention sublayer 226 of first decoder block 224-A of decoder 222 receives the encoder output of the training time series and output embeddings of the training event logs as input. The output embeddings are vector representations of the training event logs. In one embodiment, the vector representations of the training event logs may be concatenated with positional encodings to generate the output embeddings to facilitate the decoding of the training event logs temporally. Each attention head in masked multi-head self-attention sublayer 226 implements a masking mechanism for masking tokens in the output embeddings to mimic the availability of event logs at test time. In learning to attend to the right context in the event logs, each attention head would increasingly expose all tokens the event logs up to the previous step (the rest would be masked using -∞). The attention matrices from all the heads of all decoder blocks 224 are concatenated to fully represent the decoded representation of the training event logs.

Embodiments described herein are described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the providing system.

Furthermore, certain embodiments described herein are described with respect to methods and systems utilizing trained machine learning based networks (or models), as well as with respect to methods and systems for training machine learning based networks. Features, advantages or alternative embodiments herein can be assigned to the other claimed objects and vice versa. In other words, claims for methods and systems for training a machine learning based network can be improved with features described or claimed in context of the methods and systems for utilizing a trained machine learning based network, and vice versa.

In particular, the trained machine learning based networks applied in embodiments described herein can be adapted by the methods and systems for training the machine learning based networks. Furthermore, the input data of the trained machine learning based network can comprise advantageous features and embodiments of the training input data, and vice versa. Furthermore, the output data of the trained machine learning based network can comprise advantageous features and embodiments of the output training data, and vice versa.

In general, a trained machine learning based network mimics cognitive functions that humans associate with other human minds. In particular, by training based on training data, the trained machine learning based network is able to adapt to new circumstances and to detect and extrapolate patterns.

In general, parameters of a machine learning based network can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained machine learning based network can be adapted iteratively by several steps of training.

In particular, a trained machine learning based network can comprise a neural network, a support vector machine, a decision tree, and/or a Bayesian network, and/or the trained machine learning based network can be based on k-means clustering, Q-learning, genetic algorithms, and/or association rules. In particular, a neural network can be a deep neural network, a convolutional neural network, or a convolutional deep neural network. Furthermore, a neural network can be an adversarial network, a deep adversarial network and/or a generative adversarial network.

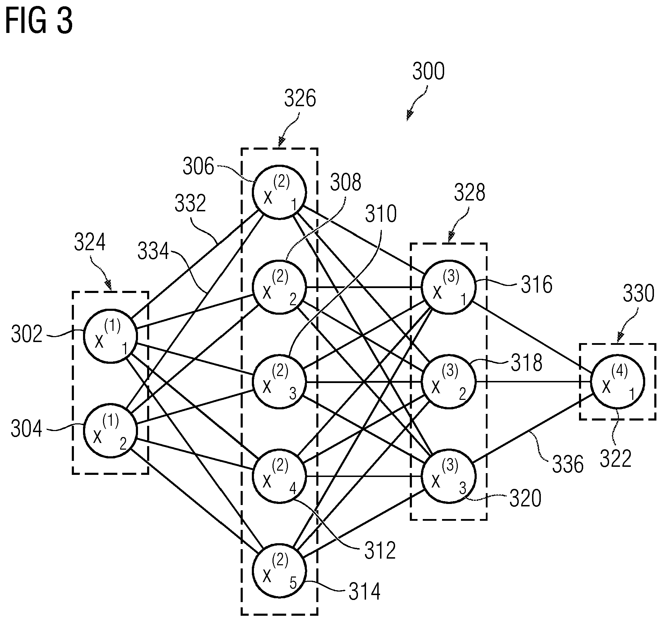

Figure 3 shows an embodiment of an artificial neural network 300, in accordance with one or more embodiments. Alternative terms for "artificial neural network" are "neural network", "artificial neural net" or "neural net". Machine learning networks described herein, such as, e.g., the transformer network of Figure 1 or transformer network 210 of Figure 2, may be implemented using artificial neural network 300.

The artificial neural network 300 comprises nodes 302-322 and edges 332, 334, ..., 336, wherein each edge 332, 334, ..., 336 is a directed connection from a first node 302-322 to a second node 302-322. In general, the first node 302-322 and the second node 302-322 are different nodes 302-322, it is also possible that the first node 302-322 and the second node 302-322 are identical. For example, in Figure 3, the edge 332 is a directed connection from the node 302 to the node 306, and the edge 334 is a directed connection from the node 304 to the node 306. An edge 332, 334, ..., 336 from a first node 302-322 to a second node 302-322 is also denoted as "ingoing edge" for the second node 302-322 and as "outgoing edge" for the first node 302-322.

In this embodiment, the nodes 302-322 of the artificial neural network 300 can be arranged in layers 324-330, wherein the layers can comprise an intrinsic order introduced by the edges 332, 334, ... , 336 between the nodes 302-322. In particular, edges 332, 334, ... , 336 can exist only between neighboring layers of nodes. In the embodiment shown in Figure 3, there is an input layer 324 comprising only nodes 302 and 304 without an incoming edge, an output layer 330 comprising only node 322 without outgoing edges, and hidden layers 326, 328 in-between the input layer 324 and the output layer 330. In general, the number of hidden layers 326, 328 can be chosen arbitrarily. The number of nodes 302 and 304 within the input layer 324 usually relates to the number of input values of the neural network 300, and the number of nodes 322 within the output layer 330 usually relates to the number of output values of the neural network 300.

In particular, a (real) number can be assigned as a value to every node 302-322 of the neural network 300. Here, x⁽ⁿ⁾ᵢ denotes the value of the i-th node 302-322 of the n-th layer 324-330. The values of the nodes 302-322 of the input layer 324 are equivalent to the input values of the neural network 300, the value of the node 322 of the output layer 330 is equivalent to the output value of the neural network 300. Furthermore, each edge 332, 334, ..., 336 can comprise a weight being a real number, in particular, the weight is a real number within the interval [-1, 1] or within the interval [0, 1]. Here, w^{(m,n)}_{i,j} denotes the weight of the edge between the i-th node 302-322 of the m-th layer 324-330 and the j-th node 302-322 of the n-th layer 324-330. Furthermore, the abbreviation w⁽ⁿ⁾_{i,j} is defined for the weight w^{(n,n+1)}_{i,j}.

In particular, to calculate the output values of the neural network 300, the input values are propagated through the neural network. In particular, the values of the nodes 302-322 of the (n+1)-th layer 324-330 can be calculated based on the values of the nodes 302-322 of the n-th layer 324-330 by ${x}_{j}^{\left(n+1\right)} = f \left({\sum }_{i} {x}_{i}^{\left(n\right)} ⋅ {w}_{i , j}^{\left(n\right)}\right) .$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 324 are given by the input of the neural network 300, wherein values of the first hidden layer 326 can be calculated based on the values of the input layer 324 of the neural network, wherein values of the second hidden layer 328 can be calculated based in the values of the first hidden layer 326, etc.

In order to set the values w^{(m,n)}_{i,j} for the edges, the neural network 300 has to be trained using training data. In particular, training data comprises training input data and training output data (denoted as tᵢ). For a training step, the neural network 300 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 300 (backpropagation algorithm). In particular, the weights are changed according to $w {′}_{i , j}^{\left(n\right)} = {w}_{i , j}^{\left(n\right)} - γ ⋅ {δ}_{j}^{\left(n\right)} ⋅ {x}_{i}^{\left(n\right)}$ wherein γ is a learning rate, and the numbers δ⁽ⁿ⁾ⱼ can be recursively calculated as ${δ}_{j}^{\left(n\right)} = \left({\sum }_{k} {δ}_{k}^{\left(n+1\right)} . {w}_{j , k}^{\left(n+1\right)}\right) ⋅ f ′ \left({\sum }_{i} {x}_{i}^{\left(n\right)} ⋅ {w}_{i , j}^{\left(n\right)}\right)$ based on δ⁽ⁿ⁺¹⁾ⱼ, if the (n+1)-th layer is not the output layer, and ${δ}_{j}^{\left(n\right)} = \left({x}_{k}^{\left(n+1\right)}-{t}_{j}^{\left(n+1\right)}\right) ⋅ f ′ \left({\sum }_{i} {x}_{i}^{\left(n\right)} ⋅ {w}_{i , j}^{\left(n\right)}\right)$ if the (n+1)-th layer is the output layer 330, wherein f' is the first derivative of the activation function, and y⁽ⁿ⁺¹⁾ⱼ is the comparison training value for the j-th node of the output layer 330.

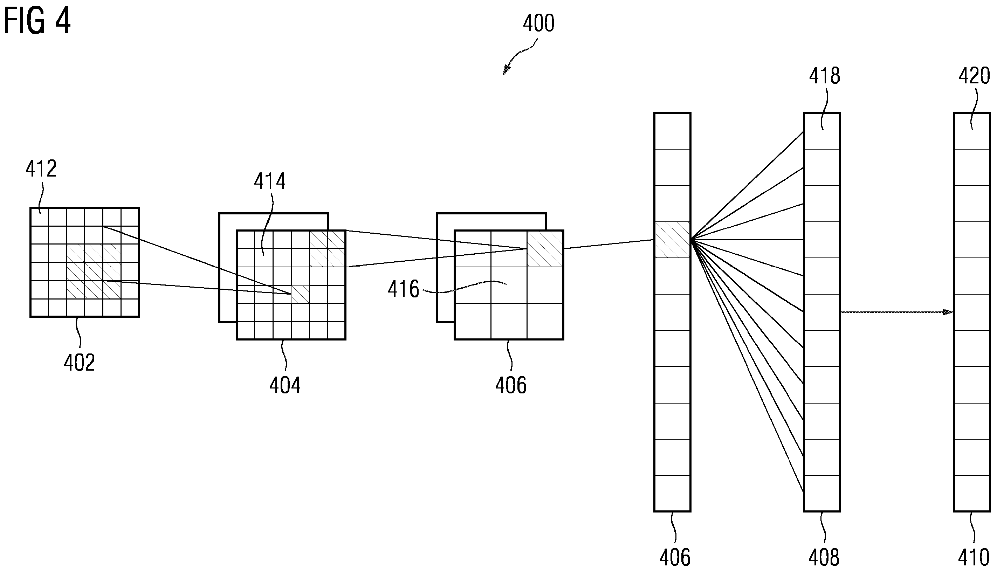

Figure 4 shows a convolutional neural network 400, in accordance with one or more embodiments. Machine learning networks described herein, such as, e.g., the transformer network of Figure 1 or transformer network 210 of Figure 2, may be implemented using convolutional neural network 400.

In the embodiment shown in Figure 4, the convolutional neural network comprises 400 an input layer 402, a convolutional layer 404, a pooling layer 406, a fully connected layer 408, and an output layer 410. Alternatively, the convolutional neural network 400 can comprise several convolutional layers 404, several pooling layers 406, and several fully connected layers 408, as well as other types of layers. The order of the layers can be chosen arbitrarily, usually fully connected layers 408 are used as the last layers before the output layer 410.

In particular, within a convolutional neural network 400, the nodes 412-420 of one layer 402-410 can be considered to be arranged as a d-dimensional matrix or as a d-dimensional image. In particular, in the two-dimensional case the value of the node 412-420 indexed with i and j in the n-th layer 402-410 can be denoted as x⁽ⁿ⁾_{[i,j]}. However, the arrangement of the nodes 412-420 of one layer 402-410 does not have an effect on the calculations executed within the convolutional neural network 400 as such, since these are given solely by the structure and the weights of the edges.

In particular, a convolutional layer 404 is characterized by the structure and the weights of the incoming edges forming a convolution operation based on a certain number of kernels. In particular, the structure and the weights of the incoming edges are chosen such that the values x⁽ⁿ⁾ₖ of the nodes 414 of the convolutional layer 404 are calculated as a convolution x⁽ⁿ⁾ₖ = Kₖ * x⁽ⁿ⁻¹⁾ based on the values x⁽ⁿ⁻¹⁾ of the nodes 412 of the preceding layer 402, where the convolution * is defined in the two-dimensional case as ${x}_{k}^{\left(n\right)} \left[i, j\right] = \left({K}_{k}*{x}^{\left(n-1\right)}\right) \left[i, j\right] = {\sum }_{i ′} {\sum }_{j ′} {K}_{k} \left[i′,j′\right] ⋅ {x}^{\left(n-1\right)} \left[i-i′, j-j′\right] .$

Here the k-th kernel Kₖ is a d-dimensional matrix (in this embodiment a two-dimensional matrix), which is usually small compared to the number of nodes 412-418 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 412-420 in the respective layer 402-410. In particular, for a convolutional layer 404, the number of nodes 414 in the convolutional layer is equivalent to the number of nodes 412 in the preceding layer 402 multiplied with the number of kernels.

If the nodes 412 of the preceding layer 402 are arranged as a d-dimensional matrix, using a plurality of kernels can be interpreted as adding a further dimension (denoted as "depth" dimension), so that the nodes 414 of the convolutional layer 404 are arranged as a (d+1)-dimensional matrix. If the nodes 412 of the preceding layer 402 are already arranged as a (d+1)-dimensional matrix comprising a depth dimension, using a plurality of kernels can be interpreted as expanding along the depth dimension, so that the nodes 414 of the convolutional layer 404 are arranged also as a (d+1)-dimensional matrix, wherein the size of the (d+1)-dimensional matrix with respect to the depth dimension is by a factor of the number of kernels larger than in the preceding layer 402.

The advantage of using convolutional layers 404 is that spatially local correlation of the input data can exploited by enforcing a local connectivity pattern between nodes of adjacent layers, in particular by each node being connected to only a small region of the nodes of the preceding layer.

In embodiment shown in Figure 4, the input layer 402 comprises 36 nodes 412, arranged as a two-dimensional 6x6 matrix. The convolutional layer 404 comprises 72 nodes 414, arranged as two two-dimensional 6x6 matrices, each of the two matrices being the result of a convolution of the values of the input layer with a kernel. Equivalently, the nodes 414 of the convolutional layer 404 can be interpreted as arranges as a three-dimensional 6x6x2 matrix, wherein the last dimension is the depth dimension.

A pooling layer 406 can be characterized by the structure and the weights of the incoming edges and the activation function of its nodes 416 forming a pooling operation based on a non-linear pooling function f. For example, in the two dimensional case the values x⁽ⁿ⁾ of the nodes 416 of the pooling layer 406 can be calculated based on the values x⁽ⁿ⁻¹⁾ of the nodes 414 of the preceding layer 404 as ${x}^{\left(n\right)} \left[i, j\right] = f \left({x}^{\left(n-1\right)}\left[{id}_{1}, {jd}_{2}\right],…, {x}^{\left(n-1\right)}\left[{id}_{1}+{d}_{1}-1, {jd}_{2}+{d}_{2}-1\right]\right)$

In other words, by using a pooling layer 406, the number of nodes 414, 416 can be reduced, by replacing a number d1·d2 of neighboring nodes 414 in the preceding layer 404 with a single node 416 being calculated as a function of the values of said number of neighboring nodes in the pooling layer. In particular, the pooling function f can be the max-function, the average or the L2-Norm. In particular, for a pooling layer 406 the weights of the incoming edges are fixed and are not modified by training.

The advantage of using a pooling layer 406 is that the number of nodes 414, 416 and the number of parameters is reduced. This leads to the amount of computation in the network being reduced and to a control of overfitting.

In the embodiment shown in Figure 4, the pooling layer 406 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 408 can be characterized by the fact that a majority, in particular, all edges between nodes 416 of the previous layer 406 and the nodes 418 of the fully-connected layer 408 are present, and wherein the weight of each of the edges can be adjusted individually.

In this embodiment, the nodes 416 of the preceding layer 406 of the fully-connected layer 408 are displayed both as two-dimensional matrices, and additionally as non-related nodes (indicated as a line of nodes, wherein the number of nodes was reduced for a better presentability). In this embodiment, the number of nodes 418 in the fully connected layer 408 is equal to the number of nodes 416 in the preceding layer 406. Alternatively, the number of nodes 416, 418 can differ.

Furthermore, in this embodiment, the values of the nodes 420 of the output layer 410 are determined by applying the Softmax function onto the values of the nodes 418 of the preceding layer 408. By applying the Softmax function, the sum the values of all nodes 420 of the output layer 410 is 1, and all values of all nodes 420 of the output layer are real numbers between 0 and 1.

A convolutional neural network 400 can also comprise a ReLU (rectified linear units) layer or activation layers with non-linear transfer functions. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer.

The input and output of different convolutional neural network blocks can be wired using summation (residual / dense neural networks), element-wise multiplication (attention) or other differentiable operators. Therefore, the convolutional neural network architecture can be nested rather than being sequential if the whole pipeline is differentiable.

In particular, convolutional neural networks 400 can be trained based on the backpropagation algorithm. For preventing overfitting, methods of regularization can be used, e.g. dropout of nodes 412-420, stochastic pooling, use of artificial data, weight decay based on the L1 or the L2 norm, or max norm constraints. Different loss functions can be combined for training the same neural network to reflect the joint training objectives. A subset of the neural network parameters can be excluded from optimization to retain the weights pretrained on another datasets.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1 or 2. Certain steps or functions of the methods and workflows described herein, including one or more of the steps or functions of Figures 1 or 2, may be performed by a server or by another processor in a network-based cloud-computing system. Certain steps or functions of the methods and workflows described herein, including one or more of the steps of Figures 1 or 2, may be performed by a client computer in a network-based cloud computing system. The steps or functions of the methods and workflows described herein, including one or more of the steps of Figures 1 or 2, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1 or 2, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 502 that may be used to implement systems, apparatus, and methods described herein is depicted in Figure 5. Computer 502 includes a processor 504 operatively coupled to a data storage device 512 and a memory 510. Processor 504 controls the overall operation of computer 502 by executing computer program instructions that define such operations. The computer program instructions may be stored in data storage device 512, or other computer readable medium, and loaded into memory 510 when execution of the computer program instructions is desired. Thus, the method and workflow steps or functions of Figures 1 or 2 can be defined by the computer program instructions stored in memory 510 and/or data storage device 512 and controlled by processor 504 executing the computer program instructions. For example, the computer program instructions can be implemented as computer executable code programmed by one skilled in the art to perform the method and workflow steps or functions of Figures 1 or 2. Accordingly, by executing the computer program instructions, the processor 504 executes the method and workflow steps or functions of Figures 1 or 2. Computer 502 may also include one or more network interfaces 506 for communicating with other devices via a network. Computer 502 may also include one or more input/output devices 508 that enable user interaction with computer 502 (e.g., display, keyboard, mouse, speakers, buttons, etc.).

Processor 504 may include both general and special purpose microprocessors, and may be the sole processor or one of multiple processors of computer 502. Processor 504 may include one or more central processing units (CPUs), for example. Processor 504, data storage device 512, and/or memory 510 may include, be supplemented by, or incorporated in, one or more application-specific integrated circuits (ASICs) and/or one or more field programmable gate arrays (FPGAs).

Data storage device 512 and memory 510 each include a tangible non-transitory computer readable storage medium. Data storage device 512, and memory 510, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 508 may include peripherals, such as a printer, scanner, display screen, etc. For example, input/output devices 508 may include a display device such as a cathode ray tube (CRT) or liquid crystal display (LCD) monitor for displaying information to the user, a keyboard, and a pointing device such as a mouse or a trackball by which the user can provide input to computer 502.

An image acquisition device 514 can be connected to the computer 502 to input image data (e.g., medical images) to the computer 502. It is possible to implement the image acquisition device 514 and the computer 502 as one device. It is also possible that the image acquisition device 514 and the computer 502 communicate wirelessly through a network. In a possible embodiment, the computer 502 can be located remotely with respect to the image acquisition device 514.

Any or all of the systems and apparatus discussed herein may be implemented using one or more computers such as computer 502.

One skilled in the art will recognize that an implementation of an actual computer or computer system may have other structures and may contain other components as well, and that Figure 5 is a high level representation of some of the components of such a computer for illustrative purposes.

Independent of the grammatical term usage, individuals with male, female or other gender identities are included within the term.

## Claims

1. A computer-implemented method (100) comprising:
receiving (102) a time series resulting from a test of a medical machine;
encoding (104) the time series using an encoder of a transformer network: generating (106) a predicted event log for the medical machine based on the encoded time series using a decoder of the transformer network, the predicted event log comprising predicted events and associated action plan recommendations; and
outputting (108) the predicted event log.

2. The computer-implemented method of claim 1, wherein the encoder comprises one or more encoder blocks each comprising a multi-head self-attention sublayer and wherein encoding the time series using an encoder of a transformer network comprises:
generating query, key, and value matrices based on a vector representation of the time series;
computing an attention matrix for each attention head of the multi-head self-attention sublayers of the one or more encoder blocks based on the query, key, and value matrices; and
combining the attention matrices for all attention heads to generate the encoded time series.

3. The computer-implemented method of claim 1, wherein the decoder comprises one or more decoder blocks each comprising a multi-head encoder-decoder attention sublayer parameterized by a key matrix and a value matrix computed based on the encoded time series and query matrix computed based on an event log embedding matrix learned during training.

4. The computer-implemented method of claim 1, further comprising training the transformer network using training event logs, wherein the decoder comprises one or more decoder blocks each comprising a masked multi-head self-attention sublayer for learning an event log embedding matrix based on the training event logs, wherein preferably the masked multi-head self-attention sublayers mask tokens in a vector representation of the training event logs.

5. The computer-implemented method of claim 1, further comprising:
preprocessing the time series to link sensors of the medical machine.

6. The computer-implemented method of claim 1, wherein the medical machine comprises at least one of a medical imaging machine or a medical laboratory machine.

7. An apparatus (502) comprising:
means for receiving (102) a time series resulting from a test of a medical machine:
means for encoding (104) the time series using an encoder of a transformer network;
means for generating (106) a predicted event log for the medical machine based on the encoded time series using a decoder of the transformer network, the predicted event log comprising predicted events and associated action plan recommendations; and
means for outputting (108) the predicted event log.

8. The apparatus of claim 7, wherein the encoder comprises one or more encoder blocks each comprising a multi-head self-attention sublayer and wherein the means for encoding the time series using an encoder of a transformer network comprises:
means for generating query, key, and value matrices based on a vector representation of the time series;
means for computing an attention matrix for each attention head of the multi-head self-attention sublayers of the one or more encoder blocks based on the query, key, and value matrices; and
means for combining the attention matrices for all attention heads to generate the encoded time series, and/or
wherein the decoder comprises one or more decoder blocks each comprising a multi-head encoder-decoder attention sublayer parameterized by a key matrix and a value matrix computed based on the encoded time series and query matrix computed based on an event log embedding matrix learned during training.

9. The apparatus of claim 7, further comprising means for training the transformer network using training event logs, wherein the decoder comprises one or more decoder blocks each comprising a masked multi-head self-attention sublayer for learning an event log embedding matrix based on the training event logs, wherein preferably the masked multi-head self-attention sublayers mask tokens in a vector representation of the training event logs .

10. The apparatus of claim 7, further comprising:
means for preprocessing the time series to link sensors of the medical machine.

11. The apparatus of claim 7, wherein the medical machine comprises at least one of a medical imaging machine or a medical laboratory machine.

12. A non-transitory computer readable medium storing computer program instructions, the computer program instructions when executed by a processor cause the processor to perform operations comprising:
receiving (102) a time series resulting from a test of a medical machine;
encoding (104) the time series using an encoder of a transformer network;
generating (106) a predicted event log for the medical machine based on the encoded time series using a decoder of the transformer network, the predicted event log comprising predicted events and associated action plan recommendations; and
outputting (108) the predicted event log.

13. The non-transitory computer readable medium of claim 12, wherein the encoder comprises one or more encoder blocks each comprising a multi-head self-attention sublayer and wherein encoding the time series using an encoder of a transformer network comprises:
generating query, key, and value matrices based on a vector representation of the time series;
computing an attention matrix for each attention head of the multi-head self-attention sublayers of the one or more encoder blocks based on the query, key, and value matrices; and
combining the attention matrices for all attention heads to generate the encoded time series, and/or
wherein the decoder comprises one or more decoder blocks each comprising a multi-head encoder-decoder attention sublayer parameterized by a key matrix and a value matrix computed based on the encoded time series and query matrix computed based on an event log embedding matrix learned during training.

14. The non-transitory computer readable medium of claim 12, further comprising training the transformer network using training event logs, wherein the decoder comprises one or more decoder blocks each comprising a multi-head self-attention sublayer for learning an event log embedding matrix based on the training event logs, wherein preferably the masked multi-head self-attention sublayers mask tokens in a vector representation of the training event logs.

15. The non-transitory computer readable medium of claim 12, the operations further comprising:
preprocessing the time series to link sensors of the medical machine.

## Patentansprüche

1. Computerimplementiertes Verfahren (100), umfassend:
Empfangen (102) einer Zeitreihe, die aus einem Test einer medizinischen Maschine resultiert;
Codieren (104) der Zeitreihe unter Verwendung eines Codierers eines Transformer-Netzwerks;
Erzeugen (106) eines Protokolls vorhergesagter Ereignisse für die medizinische Maschine basierend auf der codierten Zeitreihe unter Verwendung eines Decodierers des Transformer-Netzwerks, wobei das Protokoll vorhergesagter Ereignisse vorhergesagte Ereignisse und zugeordnete Aktionsplanempfehlungen umfasst; und Ausgeben (108) des Protokolls vorhergesagter Ereignisse.

2. Computerimplementiertes Verfahren nach Anspruch 1, wobei der Codierer einen oder mehrere Codiererblöcke umfasst, die jeweils eine Mehrkopf-Selbstaufmerksamkeits-Unterschicht umfassen, und wobei das Codieren der Zeitreihe unter Verwendung eines Codierers eines Transformer-Netzwerks umfasst: Erzeugen von Abfrage-, Schlüssel- und Wertematrizen basierend auf einer Vektordarstellung der Zeitreihe;
Berechnen einer Aufmerksamkeitsmatrix für jeden Aufmerksamkeitskopf der Mehrkopf-Selbstaufmerksamkeits-Unterschichten des einen oder der mehreren Codiererblöcke basierend auf den Abfrage-, Schlüssel- und Wertmatrizen; und Kombinieren der Aufmerksamkeitsmatrizen für alle Aufmerksamkeitsköpfe, um die codierte Zeitreihe zu erzeugen.

3. Computerimplementiertes Verfahren nach Anspruch 1, wobei der Decodierer einen oder mehrere Decodiererblöcke umfasst, die jeweils eine Mehrkopf-Codierer-Decodierer-Aufmerksamkeits-Unterschicht umfassen, die durch eine Schlüsselmatrix und eine Wertematrix, die basierend auf der codierten Zeitreihe berechnet werden, und eine Abfragematrix, die basierend auf einer während eines Trainings gelernten Ereignisprotokoll-Einbettungsmatrix berechnet wird, parametriert ist.

4. Computerimplementiertes Verfahren nach Anspruch 1, welches ferner das Trainieren des Transformer-Netzwerks unter Verwendung von Trainingsereignisprotokollen umfasst, wobei der Decodierer einen oder mehrere Decodiererblöcke umfasst, die jeweils eine maskierte Mehrkopf-Selbstaufmerksamkeits-Unterschicht zum Lernen einer Ereignisprotokoll-Einbettungsmatrix basierend auf den Trainingsereignisprotokollen umfassen, wobei vorzugsweise die maskierten Mehrkopf-Selbstaufmerksamkeits-Unterschichten Token in einer Vektordarstellung der Trainingsereignisprotokolle maskieren.

5. Computerimplementiertes Verfahren nach Anspruch 1, ferner umfassend:
Vorverarbeiten der Zeitreihe, um Sensoren der medizinischen Maschine zu verknüpfen.

6. Computerimplementiertes Verfahren nach Anspruch 1, wobei die medizinische Maschine mindestens eine medizinische Bildgebungsmaschine oder eine medizinische Labormaschine umfasst.

7. Vorrichtung (502), umfassend:
Mittel zum Empfangen (102) einer Zeitreihe, die aus einem Test einer medizinischen Maschine resultiert;
Mittel zum Codieren (104) der Zeitreihe unter Verwendung eines Codierers eines Transformer-Netzwerks;
Mittel zum Erzeugen (106) eines Protokolls vorhergesagter Ereignisse für die medizinische Maschine basierend auf der codierten Zeitreihe unter Verwendung eines Decodierers des Transformer-Netzwerks, wobei das Protokoll vorhergesagter Ereignisse vorhergesagte Ereignisse und zugeordnete Aktionsplanempfehlungen umfasst; und
Mittel zum Ausgeben (108) des Protokolls vorhergesagter Ereignisse.

8. Vorrichtung nach Anspruch 7, wobei der Codierer einen oder mehrere Codiererblöcke umfasst, die jeweils eine Mehrkopf-Selbstaufmerksamkeits-Unterschicht umfassen, und wobei die Mittel zum Codieren der Zeitreihe unter Verwendung eines Codierers eines Transformer-Netzwerks umfassen:
Mittel zum Erzeugen von Abfrage-, Schlüssel- und Wertmatrizen basierend auf einer Vektordarstellung der Zeitreihe;
Mittel zum Berechnen einer Aufmerksamkeitsmatrix für jeden Aufmerksamkeitskopf der Mehrkopf-Selbstaufmerksamkeits-Unterschichten des einen oder der mehreren Codiererblöcke basierend auf den Abfrage-, Schlüssel- und Wertmatrizen; und
Mittel zum Kombinieren der Aufmerksamkeitsmatrizen für alle Aufmerksamkeitsköpfe, um die codierte Zeitreihe zu erzeugen, und/oder
wobei der Decodierer einen oder mehrere Decodiererblöcke umfasst, die jeweils eine Mehrkopf-Codierer-Decodierer-Aufmerksamkeits-Unterschicht umfassen, die durch eine Schlüsselmatrix und eine Wertematrix, die basierend auf der codierten Zeitreihe berechnet werden, und eine Abfragematrix, die basierend auf einer während eines Trainings gelernten Ereignisprotokoll-Einbettungsmatrix berechnet wird, parametriert ist.

9. Vorrichtung nach Anspruch 7, die ferner Mittel zum Trainieren des Transformer-Netzwerks unter Verwendung von Trainingsereignisprotokollen umfasst, wobei der Decodierer einen oder mehrere Decodiererblöcke umfasst, die jeweils eine maskierte Mehrkopf-Selbstaufmerksamkeits-Unterschicht zum Lernen einer Ereignisprotokoll-Einbettungsmatrix basierend auf den Trainingsereignisprotokollen umfassen, wobei vorzugsweise die maskierten Mehrkopf-Selbstaufmerksamkeits-Unterschichten Token in einer Vektordarstellung der Trainingsereignisprotokolle maskieren.

10. Vorrichtung nach Anspruch 7, ferner umfassend:
Mittel zum Vorverarbeiten der Zeitreihe, um Sensoren der medizinischen Maschine zu verknüpfen.

11. Vorrichtung nach Anspruch 7, wobei die medizinische Maschine mindestens eine medizinische Bildgebungsmaschine oder eine medizinische Labormaschine umfasst.

12. Nichtflüchtiges computerlesbares Medium, das Computerprogrammanweisungen speichert, wobei die Computerprogrammanweisungen, wenn sie von einem Prozessor ausgeführt werden, den Prozessor veranlassen, Operationen durchzuführen, welche umfassen:
Empfangen (102) einer Zeitreihe, die aus einem Test einer medizinischen Maschine resultiert;
Codieren (104) der Zeitreihe unter Verwendung eines Codierers eines Transformer-Netzwerks;
Erzeugen (106) eines Protokolls vorhergesagter Ereignisse für die medizinische Maschine basierend auf der codierten Zeitreihe unter Verwendung eines Decodierers des Transformer-Netzwerks, wobei das Protokoll vorhergesagter Ereignisse vorhergesagte Ereignisse und zugeordnete Aktionsplanempfehlungen umfasst; und Ausgeben (108) des Protokolls vorhergesagter Ereignisse.

13. Nichtflüchtiges computerlesbares Medium nach Anspruch 12, wobei der Codierer einen oder mehrere Codiererblöcke umfasst, die jeweils eine Mehrkopf-Selbstaufmerksamkeits-Unterschicht umfassen, und wobei das Codieren der Zeitreihe unter Verwendung eines Codierers eines Transformer-Netzwerks umfasst:
Erzeugen von Abfrage-, Schlüssel- und Wertmatrizen basierend auf einer Vektordarstellung der Zeitreihe;
Berechnen einer Aufmerksamkeitsmatrix für jeden Aufmerksamkeitskopf der Mehrkopf-Selbstaufmerksamkeits-Unterschichten des einen oder der mehreren Codiererblöcke basierend auf den Abfrage-, Schlüssel- und Wertmatrizen; und
Kombinieren der Aufmerksamkeitsmatrizen für alle Aufmerksamkeitsköpfe, um die codierte Zeitreihe zu erzeugen, und/oder
wobei der Decodierer einen oder mehrere Decodiererblöcke umfasst, die jeweils eine Mehrkopf-Codierer-Decodierer-Aufmerksamkeits-Unterschicht umfassen, die durch eine Schlüsselmatrix und eine Wertematrix, die basierend auf der codierten Zeitreihe berechnet werden, und eine Abfragematrix, die basierend auf einer während eines Trainings gelernten Ereignisprotokoll-Einbettungsmatrix berechnet wird, parametriert ist.

14. Nichtflüchtiges computerlesbares Medium nach Anspruch 12, welches ferner das Trainieren des Transformer-Netzwerks unter Verwendung von Trainingsereignisprotokollen umfasst, wobei der Decodierer einen oder mehrere Decodiererblöcke umfasst, die jeweils eine Mehrkopf-Selbstaufmerksamkeits-Unterschicht zum Lernen einer Ereignisprotokoll-Einbettungsmatrix basierend auf den Trainingsereignisprotokollen umfassen, wobei vorzugsweise die maskierten Mehrkopf-Selbstaufmerksamkeits-Unterschichten Token in einer Vektordarstellung der Trainingsereignisprotokolle maskieren.

15. Nichtflüchtiges computerlesbares Speichermedium nach Anspruch 12, wobei die Operationen ferner umfassen:
Vorverarbeiten der Zeitreihe, um Sensoren der medizinischen Maschine zu verknüpfen.

## Revendications

1. Procédé (100) mis en œuvre par ordinateur, comprenant :
la réception (102) d'une série chronologique résultant d'un essai d'une machine médicale ;
le codage (104) de la série chronologique à l'aide d'un codeur d'un réseau transformeur ;
la génération (106) d'un journal d'événements prédits pour la machine médicale sur la base de la série chronologique codée à l'aide d'un décodeur du réseau transformeur, le journal d'événements prédits comprenant des événements prédits et des recommandations de plan d'action associées ; et
la fourniture en sortie (108) du journal d'événements prédits.

2. Procédé mis en œuvre par ordinateur selon la revendication 1, dans lequel le codeur comprend un ou plusieurs blocs codeurs comprenant chacun une sous-couche d'auto-attention multi-têtes et dans lequel le codage de la série chronologique au moyen d'un codeur d'un réseau transformeur comprend : la génération de matrices de requêtes, de clés et de valeurs sur la base d'une représentation vectorielle de la série chronologique ;
le calcul d'une matrice d'attention pour chaque tête d'attention des sous-couches d'auto-attention multi-têtes du ou des blocs codeurs sur la base des matrices de requêtes, de clés et de valeurs ; et
la combinaison des matrices d'attention pour toutes les têtes d'attention afin de générer la série chronologique codée.

3. Procédé mis en œuvre par ordinateur selon la revendication 1, dans lequel le décodeur comprend un ou plusieurs blocs décodeurs comprenant chacun une sous-couche d'attention codeur-décodeur multi-têtes paramétrée par une matrice de clés et une matrice de valeurs calculées sur la base de la série chronologique codée et par une matrice de requêtes calculée sur la base d'une matrice d'intégration de journal d'événements apprise pendant l'entraînement.

4. Procédé mis en œuvre par ordinateur selon la revendication 1, comprenant en outre l'entraînement du réseau transformeur à l'aide de journaux d'événements d'entraînement, dans lequel le décodeur comprend un ou plusieurs blocs décodeurs comprenant chacun une sous-couche d'auto-attention multi-têtes masquée pour l'apprentissage d'une matrice d'intégration de journal d'événements sur la base des journaux d'événements d'entraînement, dans lequel, de préférence, les sous-couches d'auto-attention multi-têtes masquées masquent des jetons dans une représentation vectorielle des journaux d'événements d'entraînement.

5. Procédé mis en œuvre par ordinateur selon la revendication 1, comprenant en outre :
le prétraitement de la série chronologique pour relier des capteurs de la machine médicale.

6. Procédé mis en œuvre par ordinateur selon la revendication 1, dans lequel la machine médicale comprend au moins l'un d'une machine d'imagerie médicale ou d'une machine de laboratoire médical.

7. Appareil (502), comprenant :
des moyens pour recevoir (102) une série chronologique résultant d'un test d'une machine médicale ;
des moyens pour coder(104) la série chronologique à l'aide d'un codeur d'un réseau transformeur ;
des moyens pour générer (106) un journal d'événements prédits pour la machine médicale sur la base de la série chronologique codée à l'aide d'un décodeur du réseau transformeur, le journal d'événements prédits comprenant des événements prédits et des recommandations de plan d'action associées ; et
des moyens pour fournir en sortie (108) le journal d'événements prédits.

8. Appareil selon la revendication 7, dans lequel le codeur comprend un ou plusieurs blocs codeurs comprenant chacun une sous-couche d'auto-attention multi-têtes et dans lequel le moyen pour coder la série chronologique à l'aide d'un codeur d'un réseau de transformateur comprend :
des moyens pour générer des matrices de requêtes, de clés et de valeurs sur la base d'une représentation vectorielle de la série chronologique ;
des moyens pour calculer une matrice d'attention pour chaque tête d'attention des sous-couches d'auto-attention multi-têtes du ou des blocs codeurs sur la base des matrices de requêtes, de clés et de valeurs ; et
des moyens pour combiner les matrices d'attention pour toutes les têtes d'attention afin de générer la série chronologique codée, et/ou
dans lequel le décodeur comprend un ou plusieurs blocs décodeurs comprenant chacun une sous-couche d'attention codeur-décodeur multi-têtes paramétrée par une matrice de clés et une matrice de valeurs calculées sur la base de la série chronologique codée et par une matrice de requêtes calculée sur la base d'une matrice d'intégration de journal d'événements apprise pendant l'entraînement.

9. Appareil selon la revendication 7, comprenant en outre des moyens pour entraîner le réseau transformeur à l'aide de journaux d'événements d'entraînement, dans lequel le décodeur comprend un ou plusieurs blocs décodeurs comprenant chacun une sous-couche d'auto-attention multi-têtes masquée pour l'apprentissage d'une matrice d'intégration de journal d'événements sur la base des journaux d'événements d'entraînement, dans lequel, de préférence, les sous-couches d'auto-attention multi-têtes masquées masquent des jetons dans une représentation vectorielle des journaux d'événements d'entraînement.

10. Appareil selon la revendication 7, comprenant en outre :
des moyens pour prétraiter la série chronologique afin de relier des capteurs de la machine médicale.

11. Appareil selon la revendication 7, dans lequel la machine médicale comprend au moins l'une d'une machine d'imagerie médicale ou d'une machine de laboratoire médical.

12. Support non transitoire lisible par ordinateur, stockant des instructions de programme informatique, les instructions de programme informatique, lors de leur exécution par un processeur, amenant le processeur à réaliser des opérations comprenant :
la réception (102) d'une série chronologique résultant d'un essai d'une machine médicale ;
le codage (104) de la série chronologique à l'aide d'un codeur d'un réseau transformeur ;
la génération (106) d'un journal d'événements prédits pour la machine médicale sur la base de la série chronologique codée à l'aide d'un décodeur du réseau transformeur, le journal d'événements prédits comprenant des événements prédits et des recommandations de plan d'action associées ; et
la fourniture en sortie (108) du journal d'événements prédits.

13. Support non transitoire lisible par ordinateur selon la revendication 12, dans lequel le codeur comprend un ou plusieurs blocs codeurs comprenant chacun une sous-couche d'auto-attention multi-têtes et dans lequel le codage de la série chronologique à l'aide d'un codeur d'un réseau transformeur comprend :
la génération de matrices de requêtes, de clés et de valeurs sur la base d'une représentation vectorielle de la série chronologique ;
le calcul d'une matrice d'attention pour chaque tête d'attention des sous-couches d'auto-attention multi-têtes du ou des blocs codeurs sur la base des matrices de requêtes, de clés et de valeurs ; et
la combinaison des matrices d'attention pour toutes les têtes d'attention afin de générer la série chronologique codée, et/ou dans lequel le décodeur comprend un ou plusieurs blocs décodeurs comprenant chacun une sous-couche d'attention codeur-décodeur multi-têtes paramétrée par une matrice de clés et une matrice de valeurs calculées sur la base de la série chronologique codée et par une matrice de requêtes calculée sur la base d'une matrice d'intégration de journal d'événements apprise pendant l'entraînement.

14. Support non transitoire lisible par ordinateur selon la revendication 12, comprenant en outre l'entraînement du réseau transformeur à l'aide de journaux d'événements d'entraînement, dans lequel le décodeur comprend un ou plusieurs blocs décodeurs comprenant chacun une sous-couche d'auto-attention multi-têtes pour l'apprentissage d'une matrice d'intégration de journal d'événements sur la base des journaux d'événements d'entraînement, dans lequel, de préférence, les sous-couches d'auto-attention multi-têtes masquées masquent des jetons dans une représentation vectorielle des journaux d'événements d'entraînement.

15. Support non transitoire lisible par ordinateur selon la revendication 12, les opérations comprenant en outre :
le prétraitement de la série chronologique pour relier des capteurs de la machine médicale.
